# EUROPEAN PATENT SPECIFICATION

(11) **EP 3 277 170 B1**
(45) Date of publication and mention of the grant of the patent: **23.01.2019**
(21) Application number: 16716390.6
(22) Date of filing: 01.04.2016
(51) Int. Cl.: A61B 5/021, A61B 5/022, A61B 5/00

(54) **TRANSDUCING PRESSURE TO A NON-INVASIVE PULSE SENSOR**
DRUCKWANDLUNG ZU EINEM NICHTINVASIVEN PULSSENSOR
TRANSDUCTION DE PRESSION À UN CAPTEUR DE PULSATIONS NON-INVASIF

(30) Priority: 02.04.2015 US 201562142389 P; 25.06.2015 US 201514750747
(43) Date of publication of application: 07.02.2018
(73) Proprietor: Microsoft Technology Licensing, LLC, Redmond, Washington 98052-6399 (US)
(72) Inventor: SAPONAS, Scott T., Redmond, Washington 98052-6399 (US); BASU, Sumit, Redmond, Washington 98052-6399 (US); MORRIS, Daniel, Redmond, Washington 98052-6399 (US); GUPTA, Sidhant, Redmond, Washington 98052-6399 (US); MALLADI, Sailaja, Redmond, Washington 98052-6399 (US); TAN, Desney S., Redmond, Washington 98052-6399 (US); VILLAR, Nicolas, Redmond, Washington 98052-6399 (US); PATEL, Shwetak N., Redmond, Washington 98052-6399 (US)
(74) Representative: CMS Cameron McKenna Nabarro Olswang LLP
(86) International application number: PCT/US2016/025456
(87) International publication number: WO 2016/161225

(56) References cited:
- JP-B2- 4 862 939
- US-A1- 2006 189 850
- US-B1- 6 491 647

## Description

### BACKGROUND

Monitoring heart rate, heart rate variability, arterial blood pressure, pulse-wave velocity, and augmentation index provide useful health information. A pulse pressure wave sensor provides a non-invasive mechanism for capturing the morphology of a pulse pressure wave which can be used in measuring heart rate, heart rate variability, arterial blood pressure, pulse-wave velocity, and augmentation index. A pulse pressure wave sensor may be incorporated into a wearable device.

US2006189850 by Philippe Cosquer et al relates to a device for measuring at least one item of physiological information in an individual, comprising a flexible membrane designed to come into contact with the skin of the individual and participating in the definition of a deformable space for a flexible substance, the substance transmitting to at least one sensor at least one physical force undergone by the membrane.

JP4862939(B2) by Shinichi Tanaka relates to a biological information measuring apparatus which can be fixed to the wrist in full contact so as to securely detect the biological information regardless of the degree of fastening a band around the wrist of a wearer, and to a wristwatch which includes a pulse detecting section which detects the pulse on the basis of a pulsatory motion of the artery near the wrist, and a band which fixes a body of the apparatus having the pulse detecting section to the wrist. Elastic sections are set on at least a part of the band so that the biological information detecting section detects the pulse when the body of the apparatus is fixed on the wrist.

US6491647 (B1) by Keith Bridger relates to a non-invasive device for measuring physiological processes. More particularly, it concerns a device that can be applied externally to the body of an animal or human to detect and quantify displacement, force, motion, vibration and acoustic effects resulting from internal biological functions. Specifically, an inexpensive device is disclosed that is compact, light, portable and comfortable, and operates satisfactorily even with imprecise location on the body, ambient noise, motion and light.

### SUMMARY

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. Furthermore, the claimed subject matter is not limited to implementations that solve any or all disadvantages noted in any part of this disclosure. Examples of embodiments of the disclosed technology are as set out in the accompanying claims. The invention is entirely defined by appended claims 1-8.

A system for transducing arterial pressure is presented. The system includes a one-piece flexible cap configured to fit around a flexible piezo-resistive sensor that is configured to alter an internal resistance upon deflection. The flexible cap includes a deflection wall shaped to conform between a radius and a flexor carpi radialis tendon, and configured to deflect towards the flexible piezo-resistive sensor in proportion to pressure applied by a radial artery. A pressure-transducing medium is sealed between the one-piece flexible cap and the flexible piezo-resistive sensor, such that deflection of the deflection wall towards the flexible piezo-resistive sensor causes proportional deflection of the flexible piezo-resistive sensor.

### BRIEF DESCRIPTION OF THE DRAWINGS

FIG. 1 shows an exploded view of a wearable electronic device.
FIG. 2 shows an example pressure transducer assembly.
FIG. 3 shows an example flexible cap that may be coupled to the pressure transducer assembly of FIG. 2.
FIG. 4A shows a pressure transducer assembly fitted with the flexible cap of FIG. 3
FIG. 4B shows a cross-section of the pressure transducer assembly of FIG. 4A.
FIG. 5A shows a wearable assembly for a pressure transducer.
FIG. 5B shows a cross section of a wrist of a user wearing the wearable assembly of FIG. 5A.
FIG. 6 illustrates an arm of a user wearing an example sensory and logic system including a pressure transducer.
FIG. 7 illustrates an arm of a user with an example pressure transduction pad worn between the arm and the pressure transducer.
FIG. 8 schematically shows a sensory-and-logic system usable to transduce a pressure wave from a radial artery to a non-invasive pulse sensor.

### DETAILED DESCRIPTION

Continuous cardiac monitoring of both healthy and unhealthy individuals may increase our understanding of heart disease, atherosclerosis, and other cardiovascular conditions, including how these conditions progress, and may thus enable early diagnosis and treatment. Pressure based sensing of the pulse wave offers non-invasive means to extract vital cardiovascular parameters, such as heart rate, arterial blood pressure, augmentation index, and pulse wave velocity.

While optical heart rate sensors have been employed in wearable devices, the signal quality is highly susceptible to noise. If the device is not worn correctly, or there is poor physical contact between the sensor and the skin, ambient light may contaminate and/or saturate the optical sensor. Further, fluid flow through the tissue associated with motion, ambulation, or pressure changes that are unrelated to blood flow will be included in the optical signal. As the optical sensor will sense light reflected off of a plurality of capillaries, the pulse peaks may be dispersed over time (wide peaks), and may only indicate one component of the pulse wave.

In contrast, pulse-pressure sensing from a single artery provides sharp pulse peaks that may indicate the entire pulse waveform, including the forward pulse wave, the reflected pulse wave, and the dicrotic notch. As a light source is not needed, a pulse-pressure sensor thus may consume less power than an optical pulse sensor. However, current non-invasive pulse-pressure sensing techniques rely on trained clinicians employing a handheld instrument on a superficial artery (e.g., radial, carotid, or femoral), and thus do not lend themselves to continuous monitoring.

In order to incorporate a pulse-pressure sensor into a wearable device, a sensor must be placed in close contact with an artery. For example, a wrist-wearable device in a watch-like format may place a pressure sensor adjacent to the radial artery. However, the pulse-pressure sensor must be able to transduce pressure from the artery through the skin and surrounding tissue. Further, the artery and sensor may move relative to one another over time. Restricting movement of the sensor and/or wrist-wearable device relative to the wearer may result in a device that is uncomfortable to wear.

A one-piece, flexible, pressure transducing cap may be used to create a conforming, bio-compatible interface between the body and the pressure transducer. The flexible cap is configured to interface with an artery of a wearer through the skin and underlying tissues, and to deflect towards the pressure transducer in proportion to blood flow and resulting pressure applied by the artery. A pressure-transducing medium is sealed between the one-piece flexible cap and the pressure transducer, such that pressure applied to the deflection wall causes proportional deflection of the pressure transducer. A pressure transducing pad optionally may be worn next to the skin over the artery, providing a distributed interface for the pressure transducer.

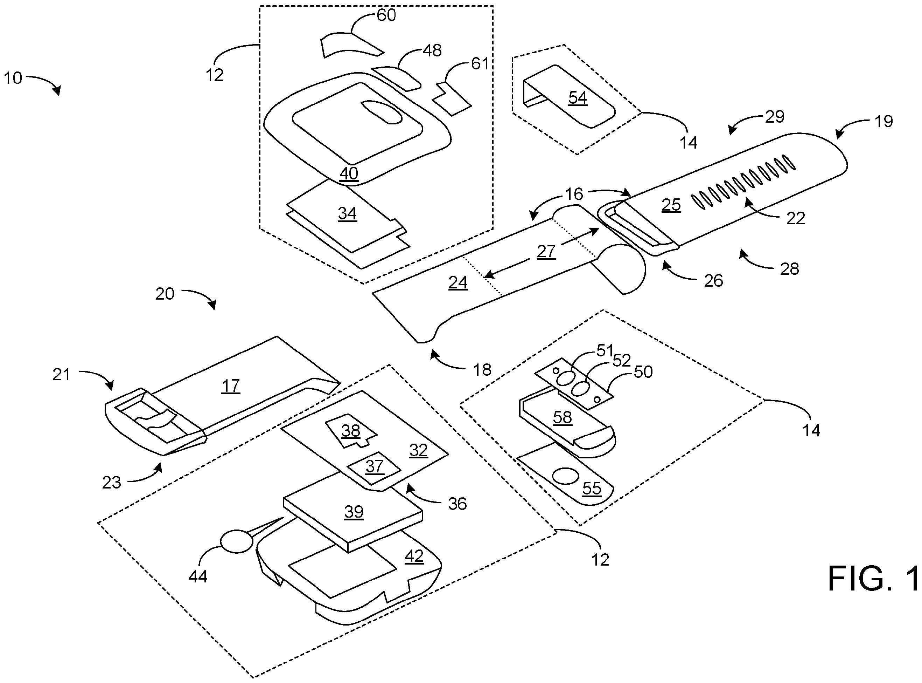

FIG. 1 shows aspects of an example sensor-and-logic system in the form of a wearable electronic device 10. The wearable electronic device 10 may be configured to measure, analyze, and/or report one or more health/fitness parameters of a wearer of wearable electronic device 10. Wearable electronic device 10 is not limiting. One or more of the features described below with reference to wearable electronic device 10 may be implemented in another sensor-and-logic system, which optionally may have a form factor that differs from wearable electronic device 10.

Wearable electronic device 10 is shown disassembled in order to depict inner componentry. The illustrated device is band-shaped and may be worn around a wrist. Wearable electronic device 10 includes a primary device 12 and a satellite device 14. Components of primary device 12 and satellite device 14 are indicated by dashed outlines. Primary device 12 may have a form function similar to the main body of a watch, and may comprise the primary user interface componentry (e.g., display, inputs, etc.) for wearable electronic device 10. Satellite device 14 may comprise pulse pressure wave transduction componentry that may enable wearable electronic device 10 to function as a wearable cardiovascular monitoring device. The accuracy of pulse pressure wave transduction may be dependent on the placement of the transduction componentry relative to the wearer's skin and underlying tissue and vasculature. For example, including the pulse pressure wave transduction componentry in satellite device 14 may enable pulse pressure wave transduction at the underside of the wearer's wrist while primary device 12 is situated on the back of the wearer's wrist in a position that is familiar to watch-wearers.

Wearable electronic device 10 is shown having a first strap 16 and a second strap 17. However, in some examples a single strap may be included, and in some examples, more than two straps may be included. The straps of wearable electronic device 10 may be elastomeric in some examples, and one or more of the straps optionally may be comprised of a conductive elastomer. First strap 16 may be connected to primary device 12 at first end 18, while second end 19 is located on the opposite, distal end of first strap 16. Similarly, second strap 17 may be connected to primary device 12 at first end 20, while second end 21 is located on the opposite, distal end of second strap 17. First strap 16 comprises primary fastening componentry 22 located towards second end 19, while second strap 17 comprises secondary fastening componentry 23 located towards second end 21. The straps and fastening componentry enable wearable electronic device 10 to be closed into a loop and to be worn on a wearer's wrist.

In this example, first strap 16 comprises a proximal portion 24 which connects to primary device 12 and a distal portion 25 that comprises primary fastening componentry 22. Proximal portion 24 and distal portion 25 may be coupled together via tertiary fastening componentry 26. In this way the distance between primary device 12 and primary fastening componentry 22 may be adjusted. However, in other examples, first strap 16 may be a single continuous strap that both connects to primary device 12 and comprises primary fastening componentry 22.

Satellite device 14 may be attached to first strap 16 at a fixed position within attachment region 27 of first strap 16, thus establishing a fixed distance between primary device 12 and satellite device 14. Primary fastening componentry 22 and secondary fastening componentry 23 are complementary, and thus may be adjustably engaged to adjust the circumference of wearable electronic device 10 without moving the fixed position of satellite device 14 relative to primary device 12. In this example, primary fastening componentry 22 includes discrete locations for engaging with secondary fastening componentry 23. However, in other examples, primary fastening componentry 22 and secondary fastening componentry 23 may be adjustably engaged along a continuous region.

Wearable electronic device 10 comprises a user-adjacent side 28 and an externally facing side 29. As such, primary device 12, satellite device 14, first strap 16, and second strap 17 may each have a user-adjacent side and externally facing side. In the closed conformation, wearable electronic device 10 thus comprises an inner surface (user-adjacent) and an outer surface (externally facing).

Wearable electronic device 10 includes various functional components integrated into primary device 12. In particular, primary device 12 includes a compute system 32, display 34, communication suite 36, and various sensors. These components draw power from one or more energy-storage cells 39. A battery--e.g., a lithium ion battery--is one type of energy-storage cell suitable for this purpose. Examples of alternative energy-storage cells include super- and ultra-capacitors. In wearable electronic devices worn on the wearer's wrist, the energy-storage cells may be curved to fit the wrist.

In general, energy-storage cells 39 may be replaceable and/or rechargeable. In some examples, recharge power may be provided through a universal serial bus (USB) port, which may include a magnetic latch to releasably secure a complementary USB connector. In other examples, the energy-storage cells 39 may be recharged by wireless inductive or ambient-light charging. In still other examples, the wearable electronic device 10 may include electro-mechanical componentry to recharge the energy-storage cells 39 from the wearer's adventitious or purposeful body motion. For example, batteries or capacitors may be charged via an electromechanical generator integrated into wearable electronic device 10. The generator may be turned by a mechanical armature that turns while the wearer is moving and wearing wearable electronic device 10.

Within primary device 12, compute system 32 is situated below display 34 and operatively coupled to display 34, along with communication suite 36, and various sensors. The compute system 32 includes a data-storage machine 37 to hold data and instructions, and a logic machine 38 to execute the instructions. Aspects of compute system 32 are described in further detail with reference to FIG. 9. These components may be situated within primary device 12 between top device housing frame 40 and bottom device housing frame 42. Primary device 12 may further comprise other actuators that may be utilized to communicate with the wearer, such as haptic motor 44, and/or a loudspeaker (not shown).

Display 34 may be any suitable type of display. In some configurations, a thin, low-power light emitting diode (LED) array or a liquid-crystal display (LCD) array may be used. An LCD array may be backlit in some implementations. In other implementations, a reflective LCD array (e.g., a liquid crystal on silicon, (LCOS) array) may be frontlit via ambient light. A curved display may also be used. Further, active-matrix organic light-emitting diode (AMOLED) displays or quantum dot displays may be used.

Communication suite 36 may include any appropriate wired or wireless communication componentry. In some examples, the communication suite 36 may include a USB port, which may be used for exchanging data between wearable electronic device 10 and other computer systems, as well as providing recharge power. The communication suite 36 may further include two-way Bluetooth, Wi-Fi, cellular, near-field communication and/or other radios. In some implementations, communication suite 36 may include an additional transceiver for optical (e.g., infrared) communication.

In wearable electronic device 10, a touch-screen sensor may be coupled to display 34 and configured to receive touch input from the wearer. The touch-screen sensor may be resistive, capacitive, or optically based. Pushbutton sensors may be used to detect the state of push button 48, which may include rockers. Input from the pushbutton sensor may be used to enact a home-key or on-off feature, control audio volume, turn a microphone on or off, *etc.*

Wearable electronic device 10 may include a plurality of additional sensors. Such sensors may include one or more microphones, visible-light sensors, ultraviolet sensors, and/or ambient temperature sensors. A microphone may provide input to compute system 32 that may be used to measure the ambient sound level or receive voice commands from the wearer. Input from the visible-light sensor, ultraviolet sensor, and ambient temperature sensor may be used to assess aspects of the wearer's environment-- i.e., the temperature, overall lighting level, and whether the wearer is indoors or outdoors.

A secondary compute system 50 is located within satellite device 14. Secondary compute system 50 may include a data-storage machine 51 to hold data and instructions, and a logic machine 52 to execute the instructions. Secondary compute system 50 may be situated between top satellite housing frame 54 and bottom satellite housing frame 55. Top satellite housing frame 54 and bottom satellite housing frame 55 may be configured to couple satellite device 14 to a fixed position within attachment region 27 on first strap 16 through the use of screws, bolts, clamps, etc. Top satellite housing frame 54 and bottom satellite housing frame 55 are shown as separate components, but in some examples, they may be coupled together by a hinge on one end, allowing satellite device 14 to be latched together around first strap 16 at the other end.

Secondary compute system 50 may be communicatively coupled to compute system 32. Satellite device 14 may mediate communication between secondary compute system 50 and compute system 32. For example, satellite device 14 may include one or more conductive contacts configured to physically intersect with one or more conductive wires extending from primary device 12 through attachment region 27 within first strap 16. In other examples, secondary compute system 50 may be coupled to compute system 32 via capacitive contact between one or more conductive contacts on satellite device 14 and one or more conductive wires within first strap 16. In other examples, a ribbon cable may extend from primary device 12 through first strap 16 such that one or more contacts on satellite device 14 can intersect with the ribbon cable when the satellite device 14 is affixed to first strap 16. In some examples, secondary compute system 50 may communicate with compute system 32 via wireless communication. In some examples, satellite device 14 may include one or more energy-storage cells. In other examples, satellite device 14 and components housed therein may draw power from energy-storage cells 39.

A pressure transducing device 58 is located within satellite device 14. When placed above the wearer's radial artery, the pressure transducing device 58 may transduce a pulse pressure wave present in the radial artery, thus functioning as a radial tonometer. The transduced pulse pressure waves may then be converted into pulse waveform signals and utilized to determine the wearer's heart rate, blood pressure, and other cardiovascular properties. Attachment region 27 may comprise a plurality of possible sensing locations, each possible sensing location having a different effective distance from primary device 12 along the first strap 16. In some examples, attachment region 27 may comprise a plurality of continuous possible sensing locations, while in other examples attachment region 27 may comprise a plurality of discrete possible sensing locations. By adjusting the distance between primary device 12 and satellite device 14, satellite device 14 and pressure transducing device 58 may be placed directly over the wearer's radial artery while primary device 12 is positioned on the back of the wearer's wrist. In some examples, satellite device 14 may be coupled to first strap 16 at a fixed position (e.g., at second end 19). In such examples, the distance between satellite device 14 and primary device 12 may be adjusted via interactions between satellite device 14 and first strap 16, via interactions between first strap 16 and primary device 12, and/or between regions of first strap 16.

Bottom satellite housing frame 55 is shown with an opening through which pressure transducing device 58 can establish contact with the wearer's wrist at the radial artery. Wearable electronic device 10 may be configured to instruct the wearer to adjust the position of satellite device 14 relative to the radial artery if a pressure detected by the pressure transducing device 58 is below a threshold, and/or if a signal quality of the transduced pressure is below a threshold. In some examples, wearable electronic device 10 may be configured to self-adjust the position of satellite device 14 and/or the overall circumference of wearable electronic device 10.

In some examples, pressure transducing device 58 may be housed and configured to interface with a wearer's wrist independently from primary device 12. For example, pressure transducing device 58 may be worn on one wrist, while primary device 12 may be worn on the other wrist. In other examples, pressure transducing device 58 may be configured to be worn while primary device 12 is not worn. Pressure transducing device 58 may thus be configured to communicate with one or more additional computing devices, (e.g., via secondary compute system 50) such as a personal computer, tablet computer, smart phone, smart watch, gaming device, etc.

FIG. 1 shows a pair of contact sensor modules 60 and 61 situated on top device housing frame 40, which may be touchable by a wearer using fingers on the hand opposite the wrist where wearable electronic device 10 is worn. In some examples, other contact sensor modules may be included in addition to or as an alternative to contact sensor modules 60 and 61. As one example, other contact modules may be attached to user-adjacent side 28 of primary device 12, first strap 16 and/or second strap 17, and thus be held in contact with points on the wearer's wrist when wearable electronic device 10 is worn. As another example, one or more contact modules may be situated at or near secondary fastening componentry 23 on the externally-facing side 29 of wearable electronic device 10 when wearable electronic device 10 is closed into a loop, thus allowing the wearer to contact a point on their body reachable with the underside of the wearer's wrist. Additionally or alternatively, one or more contact modules may be situated on the externally-facing side 29 of the loop at first strap 16 and/or second strap 17.

Contact sensor modules 60 and 61 may include independent or cooperating sensor elements, to provide a plurality of sensory functions. For example, contact sensor modules 60 and 61 may provide an electrical resistance and/or capacitance sensory function, which measures the electrical resistance and/or capacitance of the wearer's skin. Compute system 32 may use such input to assess whether or not the device is being worn, for instance. In some implementations, the sensory function may be used to determine how tightly wearable electronic device 10 is being worn. In some examples, a contact sensor module may also provide measurement of the wearer's skin temperature. In some examples, contacting multiple contact sensor modules may allow compute system 32 to determine an electrocardiogram (EKG) of the wearer.

Wearable electronic device 10 may also include motion sensing componentry, such as an accelerometer, gyroscope, and magnetometer. The accelerometer and gyroscope may furnish acceleration data along three orthogonal axes as well as rotational data about the three axes, for a combined six degrees of freedom. This sensory data can be used to provide a pedometer / calorie-counting function, for example. Data from the accelerometer and gyroscope may be combined with geomagnetic data from the magnetometer to further define the inertial and rotational data in terms of geographic orientation. The wearable electronic device 10 may also include a global positioning system (GPS) receiver for determining the wearer's geographic location and/or velocity. In some configurations, the antenna of the GPS receiver may be relatively flexible and extend into straps 16 and/or 17. In some examples, data from the motion sensing componentry may be utilized to determine a position of the wearable electronic device 10, contact modules 60 and or 61, and/or pressure transducing device 58 relative to predetermined sensing locations on the body of the device wearer.

In some examples, wearable electronic device 10 may also include one or more optical sensors paired with one or more optical sources. The optical sources may be configured to illuminate the skin and/or the underlying tissue and blood vessels of the wearer, while the optical sensors may be configured to detect illumination reflected off of the skin and/or the underlying tissue and blood vessels of the wearer. This optical data may be communicated to compute system 32, where the data may be used to determine the wearer's blood-oxygen level, pulse, blood glucose levels, or other biometric markers with optical signatures.

Compute system 32, via the sensory functions described herein, is configured to acquire various forms of information about the wearer of wearable electronic device 10. Such information must be acquired and used with utmost respect for the wearer's privacy. Accordingly, the sensory functions may be enacted subject to opt-in participation of the wearer. In implementations where personal data is collected on the device and transmitted to a remote system for processing, that data may be anonymized. In other examples, personal data may be confined to the wearable electronic device, and only non-personal, summary data transmitted to the remote system.

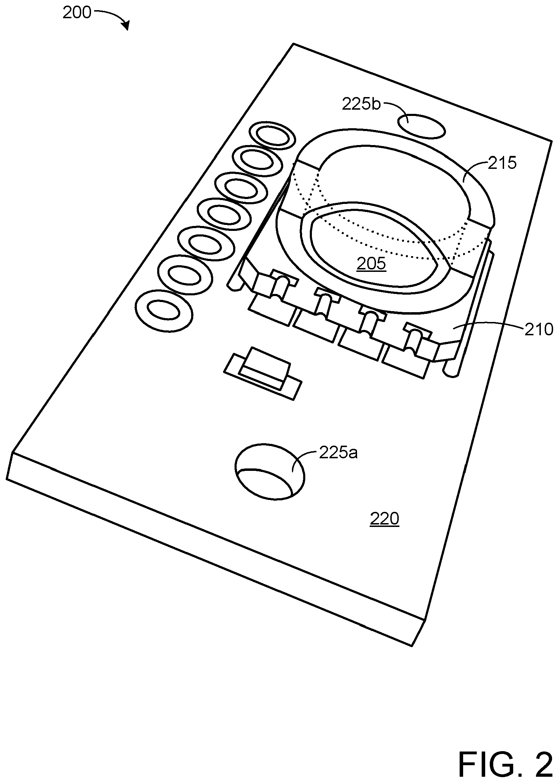

FIG. 2 shows an example pressure transducer assembly 200. Pressure transducer assembly 200 may be incorporated in a pressure transducing device for a wearable arterial pressure transducing system, such as pressure transducing device 58, as shown in FIG. 1. Pressure transducer assembly 200 may include one or more pressure transducers 205. In some examples, pressure transducer 205 may be a piezo-resistive sensor configured so that deflection of the sensor causes a change in the sensor's internal resistance. The pressure transducer may be configured as a thin membrane affixed over a deflection cavity. In this way, pressure applied to the membrane causes the membrane to deflect into the cavity. The change in resistance may then be output to a suitable analog-to-digital converter. The piezo-resistive sensor may be configured to output a signal indicative of a range of resistances corresponding to a range of absolute pressure values. For example, the piezo-resistive sensor may indicate absolute pressure values between 1 kPa and 200 kPa.

Pressure transducer 205 may be affixed to a substrate 210, which may be fabricated of ceramic, polymer, metallic, or other suitable material. An open-ended rigid conduit 215 may be affixed to the substrate surrounding the pressure transducer. Open-ended rigid conduit 215 is shown in a cut-away perspective so that pressure transducer 205 may be viewed. Open-ended rigid conduit 215 may be fabricated of metal, ceramic, or another substantially rigid, uncompressible material. Pressure changes within the conduit are thus conductively transmitted to the pressure sensor. As shown herein, open-ended rigid conduit is cylindrical. However, in some examples, pressure transducer 205 may be a non-circular shape, such as a square, oval, rectangle, etc., and thus open-ended rigid conduit 215 may be in the form of a right prism or elliptical cylinder to conform to the shape of the underlying pressure transducer.

Substrate 210, pressure transducer 205, and open-ended rigid conduit 215 may be affixed to microprocessor board 220, which may allow raw signals from pressure transducer 205 (and/or an A/D converter communicatively coupled to the pressure transducer) to be processed and/or output to other components of a pulse-pressure sensor. Microprocessor board 220 includes mounting holes 225a and 225b, which may be used to secure the pressure transducer assembly to a housing or other components. In some examples, two or more pressure transducers and accompanying open-ended rigid conduits may be affixed to the same microprocessor board. The two or more pressure transducers may be placed on the same substrate, or individual substrates on the microprocessor board.

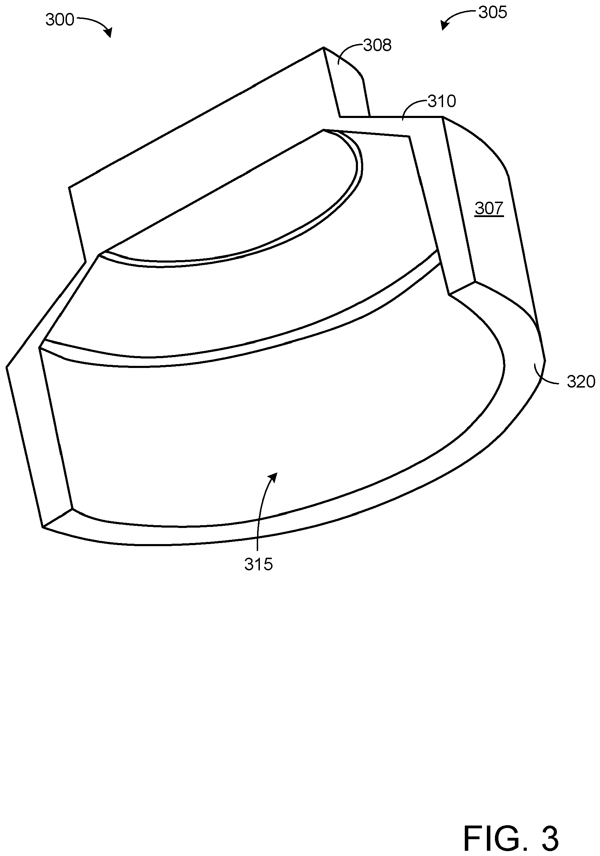

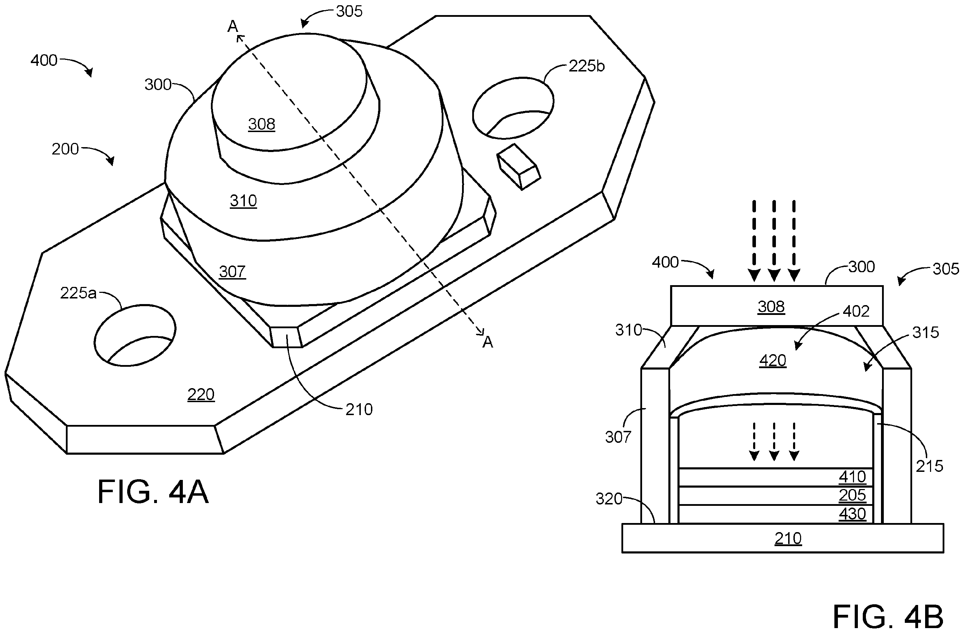

FIG. 3 shows an example flexible cap 300 that can be coupled to a pressure transducer assembly. Flexible cap 300 may be a one-piece flexible cap, for example. As shown in FIG. 4B, flexible cap 300 may be placed over open-ended rigid conduit 215, thus creating a sealed cavity over pressure transducer 205. Flexible cap 300 comprises deflection wall 305 and sidewall 307. Deflection wall 305 may cap sidewall 307, and be shaped to conform to the body of a user. Deflection wall 305 may be configured to deflect towards the flexible piezo-resistive sensor in proportion to pressure applied by an underlying artery.

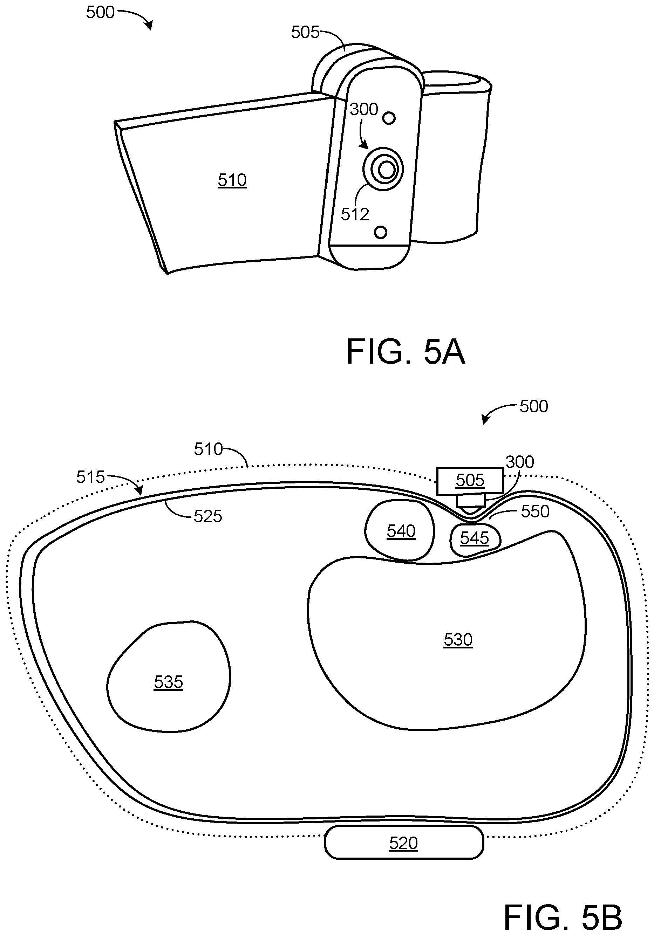

Deflection wall 305 comprises user-interfacing portion 308 and spring region 310. User-interfacing portion 308 is coupled to spring region 310, such that spring region 310 may flex up and down as variable pressure is applied to user-interfacing portion 308. Spring region 310 may bias deflection wall 305 at a maximum distance from the pressure transducer when no pressure is applied to user-interfacing portion 308. User-interfacing portion 308 may be configured to conform to tissue of a wearer so as to deflect into internal cavity 315 responsive to blood flow through an underlying artery. For example, user-interfacing portion 308 may be configured to situate between the radius and the flexor carpi radialis tendon, and thus depress into the tissue above the radial artery, applying pressure to the radial artery lumen, and thus deflecting into the sealed, internal cavity as the radial artery expands and contracts proportionate to blood flow through the radial artery. (See FIG. 5B for an example).

Spring region 310 is coupled to sidewall 307. Sidewall 307 may be configured to fit flush on the inside or outside of an open-ended rigid conduit, such as open-ended rigid conduit 215, thus conductively coupling internal cavity 315 to an underlying pressure transducer. As shown, sidewall 307 is cylindrical, but may be shaped otherwise to fit flush with the open-ended rigid conduit. Internal cavity 315 may be filled with a pressure-transducing medium. In some examples, this may be air, although in other examples, the conductive medium may be a fluid, a soft gel, a hardened gel (such as room temperature vulcanization (RTV) silicone or another silicone), a rubber, or other elastic polymer or compressible solid. The base 320 of flexible cap 300 may be secured to the substrate of the pressure transducer assembly in order to seal the pressure transducing medium within internal cavity 315.

Flexible cap 300 may be molded out of silicone, urethane, or other appropriate materials that are rigid enough to conduct pressure waves to the underlying pressure transducer, but flexible enough to conform to the skin of a wearer. In contrast, existing non-invasive pulse-pressure sensors typically utilize a solid or semi-flexible interface, which may not be conducive to constant wearing. Flexible cap 300 may be a one-piece flexible cap, fabricated from a single material. However, in some examples, user-interfacing portion 308 and spring region 310 may be fabricated from a different material than sidewall 307 and base 320. For example, user-interfacing portion 308 and spring region 310 may be fabricated from a relatively softer, more flexible material while sidewall 307 and base 320 may be fabricated from a relatively more rigid material. In some cases, flexible cap 300 may comprise a gradient of flexible materials, becoming more rigid towards base 320 and more flexible towards user-interfacing portion 308.

FIGS. 4A and 4B show an example pressure transducer assembly 400 wherein flexible cap 300 is in place surrounding open-ended rigid conduit 215 of pressure transducer assembly 200 creating a sealed cavity 402. FIG. 4B depicts a cross-section of pressure transducer assembly 400 along axis A-A. In order to seal the flexible cap to the open-ended rigid conduit or underlying substrate 210, an adhesive may be applied between base 320 and substrate 210, and/or between sidewall 307 and open-ended rigid conduit 215. For example, the adhesive may be dielectric 4207 adhesive from Dow Corning or another suitable adhesive. Additionally or alternatively, an O-ring may be used to secure the flexible cap to the open-ended rigid conduit. The O-ring may be secured around the flexible cap using an adhesive and/or via pressure from a surrounding enclosure. In some examples, the flexible cap may be affixed through welding, melting, or soldering, and/or may be secured via screws or clamps.

The interior sidewall 307 of the flexible cap and the exterior sidewall of open-ended rigid conduit 215 may be complementarily configured to allow for the flexible cap to screw on to the open-ended rigid conduit, to secure into place via a barb/ridge tandem, or slot/tab tandems. Similarly, base 320 of the flexible cap and the surface of substrate 210 may be complementarily configured to allow all or part of base 320 to securely insert into the substrate.

A gel layer 410, such as a thing gel film, may be placed over the pressure transducer on a first side of the transducer in order to protect the transducer from pressure from conductive medium 420. Pressure transducer 205 is shown situated with a deflection cavity 430 on the second, opposite side of the transducer. As pressure is applied to user-interfacing portion 308, spring region 310 deflects towards pressure transducer 205, thereby compressing the conductive medium 420. The conductive medium thus transfers the pressure to pressure transducer 205 via gel layer 410, allowing pressure transducer 205 to deflect into deflection cavity 430, thus changing the internal resistance of pressure transducer 205. As shown in FIG. 4B the user-interfacing portion is perpendicular to the sidewall. In some examples, the exterior side of user-interfacing portion 308 may be domed, or otherwise configured to conform to the wrist of the user. User-interfacing portion 308 may have a greater thickness than spring region 310, or the entire deflection wall may be of a single thickness. Spring region 310 may be angled with respect to the flexible piezo-resistive sensor. As shown, spring region 310 may decrease in diameter from the side wall to the user-interfacing portion. In other examples, spring region 310 may comprise a series of accordion-like folds.

As described with regard to FIG. 3, conductive medium 420 may be a liquid, solid, gel, or gas. As flexible cap 300 is sealed to pressure transducer assembly 200, thus confining conductive medium 420 to sealed cavity 402, the conductive medium does not need to be a biocompatible or sweat-resistant substance. The conductive medium may be selected based on the application of the pressure transducer. Solid or hardened gel mediums may be good mechanical conductors that yield a high signal to noise ratio. Air or other gaseous mediums may provide excellent buffering, thus reducing the risk of damaging the transducer. Liquid or soft gel mediums may have sufficient conduction while providing a cushion against damaging the transducer.

FIG. 5A shows a wearable assembly 500 for a pressure transducer. Wearable assembly 500 includes a satellite housing 505 wherein a pressure transducer assembly may be housed. Satellite housing 505 is coupled to strap 510, which may be used to secure the wearable assembly around a wrist of a user. Satellite housing 505 includes an opening 512 through which all or part of flexible cap 300 may protrude.

FIG. 5B shows a cross section of wearable assembly 500 coupled to a wrist 515 of a user. In this example, wearable assembly 500 further includes primary device 520. In this configuration, satellite housing 505 and its internal components, including a pressure transducer assembly may be the functional equivalent of satellite device 14 described with reference to FIG. 1, while primary device 520 may be the functional equivalent of primary device 12 described with reference to FIG. 1.

Illustrated components of wrist 515 include skin 525, radius 530, ulna 535, flexor carpi radialis tendon 540, radial artery 545, and tissue 550. Satellite housing 505 may be placed such that flexible cap 300 depresses the skin of the wearer into tissue 550 between tendon 540 and radius 530, thus compressing the lumen of the radial artery. In this position, pressure pulse waves in the radial artery may apply a pressure to flexible cap 300, which may be mechanically conducted to the underlying pressure transducer via the pressure-transducing medium.

Despite the pliability of flexible cap 300, achieving the necessary contact to couple the pressure transducer to the radial artery is dependent on the anatomy of the individual wearing the wearable assembly. The size and location of the radius and the flexor carpi radialis tendon vary from person to person, and thus the dimensions of the arterial lumen vary from person to person. For some users, a relatively rigid assembly will be capable of fitting comfortably in the arterial lumen, but for others, the flexible cap will primarily contact the skin above the radius or tendon. As such, a wearable assembly may be provided with an assortment of flexible caps including user-interfacing portions of varying sizes and/or shapes. This may allow a user to select the cap that best conforms to their anatomy. In some examples, a flexible cap may be molded to a user's specifications. A user's arm may be imaged with depth camera, laser scanner, magnetic resonance imager, or other three-dimensional imaging device. A custom flexible cap may then be designed and molded or printed with a three-dimensional printer, as an example.

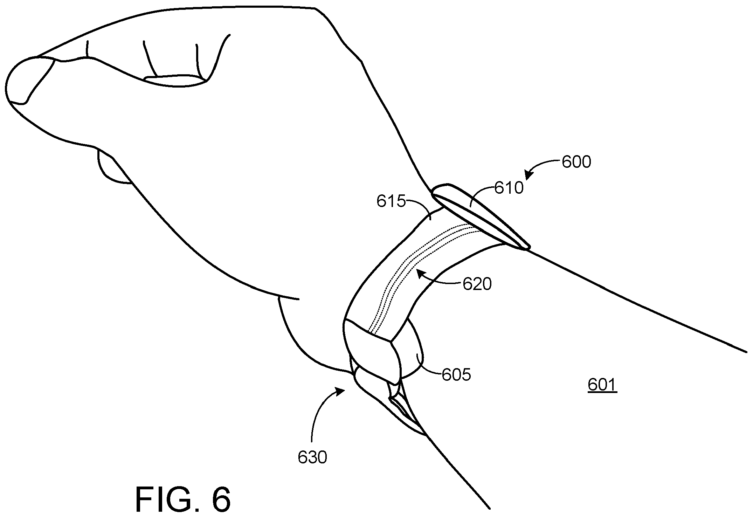

FIG. 6 depicts an example wearable electronic device 600 coupled to the wrist of a wearer 601. Wearable electronic device 600 includes a satellite device 605 which houses a pressure transducer assembly, as well as primary device 610. Satellite device 605 and primary device 610 are physically coupled to each other and to the wrist of wearer 601 via strap 615. Satellite device 605 and primary device 610 are communicatively coupled to one another via conductors 620 within strap 615.

In this example, satellite device 605 includes a fluid filled bladder 630 which is placed in contact with the skin of wearer 601. In this way, the fluid filled bladder may dynamically take the shape of the arterial lumen. The fluid within the bladder may thus act as the pressure transducing medium between the radial artery and an included pressure transducer.

In some examples, the pressure transducer may be located within the fluid filled bladder in direct contact with the fluid (and/or separated by a thin gel layer). However, the use of the bladder may also allow for the pressure transducer to be decoupled from the site of contact between the bladder and the radial artery. For example, the fluid filled bladder may be placed in contact with the arterial lumen, while the pressure transducer is coupled to the bladder by a small rigid tube. For example, the bladder may be housed within satellite device 605 while the pressure transducer is housed in the primary device 610. The use of a bladder and potential increase in the size of the interface may yield a signal that contains additional noise and motion artifacts. As such, motion may be filtered from the signal based on a signal from a motion sensor (e.g., accelerometer, gyroscope) included in the wearable assembly prior to determining cardiovascular metrics from the pressure signal.

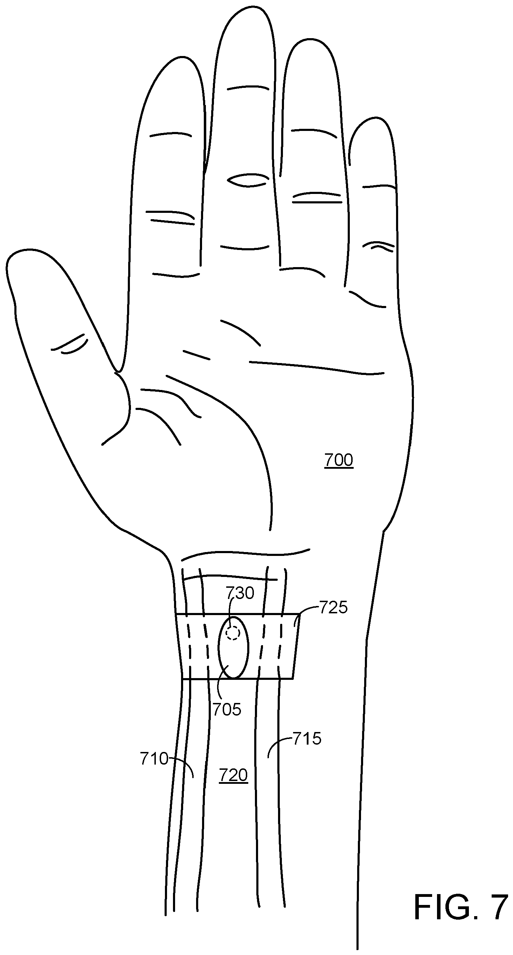

FIG. 7 illustrates an arm of a user 700 with an example pressure transducing pad 705 that may be placed between the arm and the pressure transducer. Pressure transducing pad 705 may be temporarily attached to skin of user 700 between the radius 710 and flexor carpi radialis tendon 715. The user may detect their pulse with a finger, for example, and then lay the pressure transducing pad over the location of the detected pulse. Pressure transducing pad 705 may be fabricated from silicon, polyurethane, flexible foams, or other flexible materials. In this example, the pressure transducing pad is placed parallel to the radial bone in the arterial lumen 720. Pressure transducing pad 705 may then be secured in place with a temporary adhesive. In this example, an adhesive tape 725 adheres the pressure transducing pad in place, but in some examples, the pressure transducing pad may be self-adhering, for example, with an adhesive on the base of the pad, or integrated into a bandage-style form. The flexible cap of the pressure transducer assembly may then be placed in contact with the pressure transducing pad (e.g., at 730). In this way, the user does not need to place the flexible cap as precisely as they would without the pad, as the pressure transducing pad has a greater surface area than the user-interfacing portion. The pressure transducing pad may be configured to deflect outwards from the skin proportionate to pressure applied by a radial artery such that outward deflection of the pressure transducing pad causes proportional deflection of the deflection wall of the flexible cap towards an underlying flexible piezo-resistive sensor when the user-interfacing portion is placed in contact with the pressure transducing pad.

Further, the use of a pressure transducing pad allows for integration (and thus amplification) of the arterial pulse pressure over a larger area. For individuals with a pulse that is weak or difficult to locate, this aids in finding and attaining a strong pulse signal. Additionally, the larger interaction area may increase comfort for the user, and decrease skin marking. As the pressure transducing pad has a greater surface area than the flexible cap, some movement of the flexible cap may be tolerated without losing or diminishing the quality of the pressure signal. As per the flexible cap, the pressure transducing pad may be provided in a variety of shapes and sizes, or may be custom designed for an individual user. In this way, the pressure transducing pad may be fit between the radius and tendon. Otherwise, the signal may be dampened and subject to additional noise.

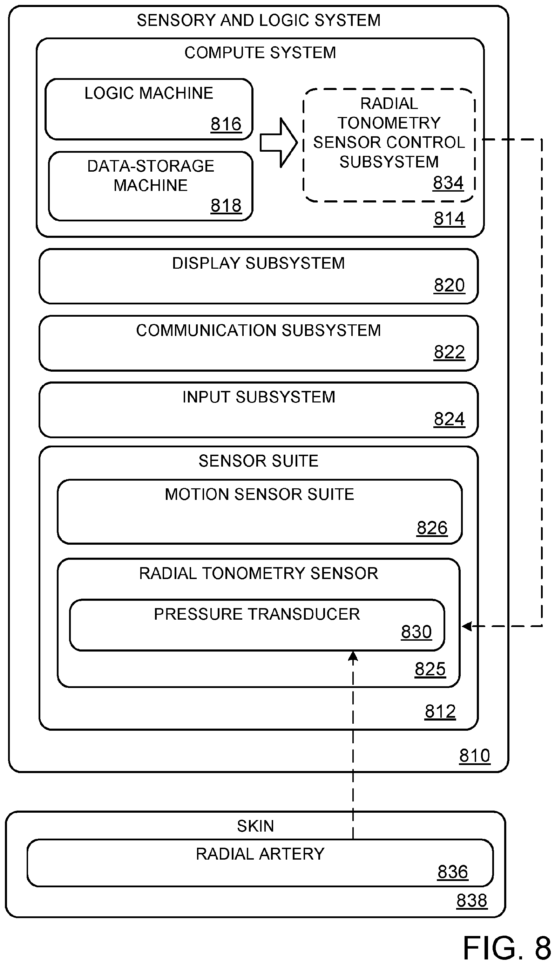

As evident from the foregoing description, the methods and processes described herein may be tied to a sensory-and-logic system of one or more machines. Such methods and processes may be implemented as a computer-application program or service, an application-programming interface (API), a library, firmware, and/or other computer-program product. FIG. 1 shows one, non-limiting example of a sensory-and-logic system to enact the methods and processes described herein. However, these methods and process may also be enacted on sensory-and-logic systems of other configurations and form factors, as shown schematically in FIG. 8.

FIG. 8 schematically shows a form-agnostic sensory-and-logic system 810 that includes a sensor suite 812 operatively coupled to a compute system 814. The compute system includes a logic machine 816 and a data-storage machine 818. The compute system is operatively coupled to a display subsystem 820, a communication subsystem 822, an input subsystem 824, and/or other components not shown in FIG. 8.

Logic machine 816 includes one or more physical devices configured to execute instructions. The logic machine may be configured to execute instructions that are part of one or more applications, services, programs, routines, libraries, objects, components, data structures, or other logical constructs. Such instructions may be implemented to perform a task, implement a data type, transform the state of one or more components, achieve a technical effect, or otherwise arrive at a desired result.

Logic machine 816 may include one or more processors configured to execute software instructions. Additionally or alternatively, the logic machine may include one or more hardware or firmware logic machines configured to execute hardware or firmware instructions. Processors of the logic machine may be single-core or multi-core, and the instructions executed thereon may be configured for sequential, parallel, and/or distributed processing. Individual components of a logic machine optionally may be distributed among two or more separate devices, which may be remotely located and/or configured for coordinated processing. Aspects of a logic machine may be virtualized and executed by remotely accessible, networked computing devices in a cloud-computing configuration.

Data-storage machine 818 includes one or more physical devices configured to hold instructions executable by logic machine 816 to implement the methods and processes described herein. When such methods and processes are implemented, the state of the data-storage machine may be transformed-e.g., to hold different data. The data-storage machine may include removable and/or built-in devices; it may include optical memory (e.g., CD, DVD, HD-DVD, Blu-Ray Disc, etc.), semiconductor memory (e.g., RAM, EPROM, EEPROM, etc.), and/or magnetic memory (e.g., hard-disk drive, floppy-disk drive, tape drive, MRAM, etc.), among others. The data-storage machine may include volatile, nonvolatile, dynamic, static, read/write, read-only, random-access, sequential-access, location-addressable, file-addressable, and/or content-addressable devices.

Data-storage machine 818 includes one or more physical devices. However, aspects of the instructions described herein alternatively may be propagated by a communication medium (e.g., an electromagnetic signal, an optical signal, etc.) that is not held by a physical device for a finite duration.

Aspects of logic machine 816 and data-storage machine 818 may be integrated together into one or more hardware-logic components. Such hardware-logic components may include field-programmable gate arrays (FPGAs), program- and application-specific integrated circuits (PASIC / ASICs), program- and application-specific standard products (PSSP / ASSPs), system-on-a-chip (SOC), and complex programmable logic devices (CPLDs), for example.

Display subsystem 820 may be used to present a visual representation of data held by data-storage machine 818. This visual representation may take the form of a graphical user interface (GUI). As the herein described methods and processes change the data held by the storage machine, and thus transform the state of the storage machine, the state of display subsystem 820 may likewise be transformed to visually represent changes in the underlying data. Display subsystem 820 may include one or more display subsystem devices utilizing virtually any type of technology. Such display subsystem devices may be combined with logic machine 816 and/or data-storage machine 818 in a shared enclosure, or such display subsystem devices may be peripheral display subsystem devices. Display 34 of FIG. 1 is an example of display subsystem 820.

Communication subsystem 822 may be configured to communicatively couple compute system 814 to one or more other computing devices. The communication subsystem may include wired and/or wireless communication devices compatible with one or more different communication protocols. As non-limiting examples, the communication subsystem may be configured for communication via a wireless telephone network, a local- or wide-area network, and/or the Internet. Communication suite 36 of FIG. 1 is an example of communication subsystem 822.

Input subsystem 824 may comprise or interface with one or more user-input devices such as a keyboard, touch screen, button, dial, joystick, or switch. In some embodiments, the input subsystem may comprise or interface with selected natural user input (NUI) componentry. Such componentry may be integrated or peripheral, and the transduction and/or processing of input actions may be handled on- or off-board. Example NUI componentry may include a microphone for speech and/or voice recognition; an infrared, color, stereoscopic, and/or depth camera for machine vision and/or gesture recognition; a head tracker, eye tracker, accelerometer, and/or gyroscope for motion detection and/or intent recognition. Push button 48 of FIG. 1 is an example of input subsystem 824.

Sensor suite 812 may include one or more different sensors-*e.g*., radial tonometry sensor 825, a touch-screen sensor, push-button sensor, microphone, visible-light sensor, ultraviolet sensor, ambient-temperature sensor, contact sensors, and/or GPS receiver-as described above with reference to FIG. 1. Sensor suite 812 may include motion sensor suite 826. Motion sensor suite 826 may include one or more of an accelerometer, gyroscope, magnetometer, or other suitable motion detectors.

As described herein, radial tonometry sensor 825 may include pressure transducer 830. Compute system 814 may include radial tonometry control subsystem 834, which may be communicatively coupled to logic machine 816 and data-storage machine 818. Pressure transducer 830 may comprise one or more piezo-resistive sensors configured to provide absolute pressure signals to compute system 814 via an analog-to-digital converter. Pressure transducer 830 may be configured to transduce pressure waves from the radial artery 836 through the skin 838 of the user.

Radial tonometry control subsystem 834 may further process the raw signals to determine heart rate, blood pressure, caloric expenditures, etc. Processed signals may be stored and output via compute system 814. Control signals sent to radial tonometry sensor 825 may be based on signals received from pressure transducer 830, signals derived from sensor suite 812, information stored in data-storage machine 818, input received from communication subsystem 822, input received from input subsystem 824, etc.

In an example, a system for transducing arterial pressure comprises: a flexible piezo-resistive sensor configured to alter an internal resistance as a function of deflection of the flexible piezo-resistive sensor; a one-piece flexible cap including: a sidewall sized to fit around the flexible piezo-resistive sensor, and a deflection wall capping the sidewall and shaped to conform between a radius and a flexor carpi radialis tendon, the deflection wall configured to deflect towards the flexible piezo-resistive sensor in proportion to pressure applied by a radial artery; and a pressure-transducing medium sealed between the one-piece flexible cap and the flexible piezo-resistive sensor such that deflection of the deflection wall towards the flexible piezo-resistive sensor causes proportional deflection of the flexible piezo-resistive sensor. In this example or any other example, the deflection wall includes a spring region between a user-interfacing portion of the deflection wall at a maximum distance from the flexible piezo-resistive sensor when no pressure is applied to the user-interfacing portion. In this example or any other example, the user-interfacing portion is perpendicular to the sidewall. In this example or any other example, the spring region is angled with respect to the flexible piezo-resistive sensor and decreases in diameter from the sidewall to the user-interfacing portion. In this example or any other example, the user-interfacing portion has a greater thickness than the spring region. In this example or any other example, the flexible piezo-resistive sensor is affixed to a substrate, and wherein the system further comprises: an open-ended rigid conduit affixed to the substrate surrounding the flexible piezo-resistive sensor, the open-ended rigid conduit configured to fit flush within the sidewall. In this example or any other example, the open-ended rigid conduit and sidewall are cylindrical. In this example or any other example, the flexible piezo-resistive sensor is affixed to the substrate such that the flexible piezo-resistive sensor deflects into a deflection cavity responsive to pressure applied to the user-interfacing portion. In this example or any other example, the system further comprises a pressure transducing pad temporarily attachable to the skin of a user between a radius of the user and a flexor carpi radialis tendon of the user and having a greater surface area than the user-interfacing portion of the deflection wall, the pressure transducing pad configured to deflect outwards from the skin proportionate to pressure applied by a radial artery such that outward deflection of the pressure transducing pad causes proportional deflection of the deflection wall towards the flexible piezo-resistive sensor when the user-interfacing portion is placed in contact with the pressure transducing pad.

In an example, a system for detecting arterial pressure comprises: a flexible piezo-resistive sensor configured to alter an internal resistance responsive to pressure applied to a first side of the flexible piezo-resistive sensor; a fixed quantity of a pressure-transducing medium located in a sealed cavity on the first side of the flexible piezo-resistive sensor and configured to transmit pressure changes to the flexible piezo-resistive sensor; and a flexible cap surrounding the sealed cavity, the flexible cap configured to conform to tissue of a user so as to deflect into the sealed cavity responsive to blood flow through an underlying artery, thereby applying a pressure to the pressure-transducing medium proportionate to the arterial blood flow. In this example or any other example, the flexible cap comprises: a sidewall sized to fit around the flexible piezo-resistive sensor, and a deflection wall capping the sidewall and shaped to conform between a radius and a flexor carpi radialis tendon, the deflection wall configured to deflect towards the flexible piezo-resistive sensor in proportion to pressure changes in a radial artery. In this example or any other example, the deflection wall includes a spring region between a user-interfacing portion of the deflection wall and the sidewall, the spring region biasing the deflection wall at a maximum distance from the flexible piezo-resistive sensor when no pressure is applied to the user-interfacing portion. In this example or any other example, the spring region is angled with respect to the flexible piezo-resistive sensor and decreases in diameter from the sidewall to the user-interfacing portion. In this example or any other example, the user-interfacing portion is perpendicular to the sidewall. In this example or any other example, the system further comprises: a substrate affixed to a second, opposite side of the flexible piezo-resistive sensor; and an open-ended rigid conduit affixed to the substrate surrounding the flexible piezo-resistive sensor, the open-ended rigid conduit configured to fit flush within the sidewall. In this example or any other example, the flexible piezo-resistive sensor is affixed to the substrate such that pressure applied to the flexible piezo-resistive sensor causes the flexible piezo-resistive sensor to deflect into a deflection cavity proportionate to the applied pressure, the deflection cavity located on the second side of the flexible piezo-resistive sensor. In this example or any other example, the system further comprises: a pressure transducing pad temporarily attachable to skin of a user between a radius of the user and a flexor carpi radialis tendon of the user and having a greater surface area than the user-interfacing portion of the deflection wall, the pressure transducing pad configured to deflect outwards from the skin proportionate to pressure applied by a radial artery such that outward deflection of the pressure transducing pad causes proportional pressure to be applied to the deflection wall when the user-interfacing portion is in contact with the pressure transducing pad. In this example or any other example, the system further comprises: a gel layer located on the first side of the flexible piezo-resistive sensor between the flexible piezo-resistive sensor and the pressure transducing medium. In this example or any other example, the flexible cap is a one-piece flexible cap.

In an example, a wearable assembly for a radial artery tonometry sensor system comprises: an adjustable strap configured to secure the radial artery tonometry sensor system to a wrist of a user; a satellite housing connected to the adjustable strap; and a pressure transducing assembly located within the satellite housing, and comprising: a flexible piezo-resistive sensor configured to alter an internal resistance as a function of deflection of the flexible piezo-resistive sensor; a one-piece flexible cap configured to at least partially protrude through an opening in the satellite housing, and including: a sidewall sized to fit around the flexible piezo-resistive sensor, and a deflection wall capping the sidewall and shaped to conform between a radius and a flexor carpi radialis tendon, the deflection wall configured to deflect towards the flexible piezo-resistive sensor in proportion to pressure applied by a radial artery; and a pressure-transducing medium sealed between the one-piece flexible cap and the flexible piezo-resistive sensor such that deflection of the deflection wall towards the flexible piezo-resistive sensor causes proportional deflection of the flexible piezo-resistive sensor.

The configurations and approaches described herein are exemplary in nature, and that these specific implementations or examples are not to be taken in a limiting sense, because numerous variations are feasible. The specific routines or methods described herein may represent one or more processing strategies. As such, various acts shown or described may be performed in the sequence shown or described, in other sequences, in parallel, or omitted.

The invention is entirely defined by appended claims 1-8.

## Claims

1. A system (200) for transducing arterial pressure, comprising:
a flexible piezo-resistive sensor (205) configured to alter an internal resistance as a function of deflection of the flexible piezo-resistive sensor (205), wherein the flexible piezo-resistive sensor (205) is affixed to a substrate (210) such that the flexible piezo-resistive sensor (205) deflects into a deflection cavity (315, 402) responsive to pressure applied to a user-interfacing portion (308);
a one-piece flexible cap (300) including:
a sidewall (307) sized to fit around the flexible piezo-resistive sensor (205), and
a deflection wall (305) capping the sidewall and shaped to conform between a radius and a flexor carpi radialis tendon, the deflection wall (305) configured to deflect towards the flexible piezo-resistive sensor (205) in proportion to pressure applied by a radial artery, wherein the deflection wall (305) includes a spring region (310) between a user-interfacing portion (308) of the deflection wall (305) and the sidewall (307), the spring region (310) biasing the deflection wall (307) at a maximum distance from the flexible piezo-resistive sensor (205) when no pressure is applied to the user-interfacing portion (308);
a pressure-transducing medium sealed between the one-piece flexible cap (300) and the flexible piezo-resistive sensor (205) such that deflection of the deflection wall (305) towards the flexible piezo-resistive sensor (205) causes proportional deflection of the flexible piezo-resistive sensor (205); and
an open-ended rigid conduit (215) affixed to the substrate (210) surrounding the flexible piezo-resistive sensor (205), the open-ended rigid conduit (215) configured to fit flush within the sidewall (307).

2. The system of claim 1, wherein the user-interfacing portion (308) is perpendicular to the sidewall (307).

3. The system of claim 2, wherein the spring region (310) is angled with respect to the flexible piezo-resistive sensor (205) and decreases in diameter from the sidewall (307) to the user-interfacing portion (308).

4. The system of claim 1, further comprising:
a pressure transducing pad (705) temporarily attachable to skin of a user between a radius of the user and a flexor carpi radialis tendon of the user and having a greater surface area than the user-interfacing portion (310) of the deflection wall (305), the pressure transducing pad (705) configured to deflect outwards from the skin proportionate to pressure applied by a radial artery such that outward deflection of the pressure transducing pad (705) causes proportional deflection of the deflection wall (305) towards the flexible piezo-resistive sensor (205) when the user-interfacing portion (308) is placed in contact with the pressure transducing pad (705).

5. A method for detecting arterial pressure, comprising:
applying a flexible cap (300) to tissue of a user above an underlying artery such that the flexible cap (300) deflects into a sealed cavity (402) responsive to blood flow through the underlying artery;
via the flexible cap (300), applying a pressure proportionate to the arterial blood flow to a fixed quantity of a pressure-transducing medium located in the sealed cavity (402) on a first side of a flexible piezo-resistive sensor (215); and
via the pressure transducing medium, transmitting pressure changes to the flexible piezo-resistive sensor (205), the flexible piezo-resistive sensor (205) configured to alter an internal resistance responsive to pressure applied to the first side of the flexible piezo-resistive sensor (205), wherein the flexible cap (300) comprises:
a sidewall (307) sized to fit around the flexible piezo-resistive sensor (205), and
a deflection wall (305) capping the sidewall (307) and shaped to conform between a radius and a flexor carpi radialis tendon, the deflection wall (305) configured to deflect towards the flexible piezo-resistive sensor (205) in proportion to pressure changes in a radial artery, wherein the deflection wall (305) includes a spring region (310) between a user-interfacing portion (308) of the deflection wall (305) and the sidewall (307), the spring region (310) biasing the deflection wall (305) at a maximum distance from the flexible piezo-resistive sensor (205) when no pressure is applied to the user-interfacing portion (308),
wherein a substrate (210) is affixed to a second, opposite side of the flexible piezo-resistive sensor (205), and wherein an open-ended rigid conduit (215) is affixed to the substrate ()210) surrounding the flexible piezo-resistive sensor (205) such that pressure applied to the flexible piezo-resistive sensor (205) causes the flexible piezo-resistive sensor (205) to deflect into a deflection cavity (315,402) proportionate to the applied pressure, the deflection cavity (315,402) located on the second side of the flexible piezo-resistive sensor (205), the open-ended rigid conduit (315) configured to fit flush within the sidewall (307).

6. The method of claim 5, wherein the spring region (310) is angled with respect to the flexible piezo-resistive sensor (205) and decreases in diameter from the sidewall (307) to the user-interfacing portion (310).

7. The method of claim 5, wherein the user-interfacing portion (308) is perpendicular to the sidewall (307).

8. The method of claim 5, further comprising:
a pressure transducing pad (705) temporarily attachable to skin of a user between a radius of the user and a flexor carpi radialis tendon of the user and having a greater surface area than the user-interfacing portion of the deflection wall (305), the pressure transducing pad (705) configured to deflect outwards from the skin proportionate to pressure applied by a radial artery such that outward deflection of the pressure transducing pad (705) causes proportional pressure to be applied to the deflection wall (305) when the user-interfacing portion (310) is in contact with the pressure transducing pad (705).

## Patentansprüche

1. System (200) zur arteriellen Druckumwandlung, umfassend:
einen flexiblen piezoresistiven Sensor (205), der zum Ändern eines Innenwiderstands in Abhängigkeit von der Ablenkung des flexiblen piezoresistiven Sensors (205) konfiguriert ist, wobei der flexible piezoresistive Sensor (205) an einem Substrat (210) befestigt ist, sodass sich der flexible piezoresistive Sensor (205) in Reaktion auf den Druck, der an einen Benutzerschnittstellenabschnitt (308) angelegt wird, in einen Auslenkungshohlraum (315, 402) auslenkt;
eine einstückige flexible Kappe (300), die aufweist:
eine Seitenwand (307), die bemessen ist, um um den flexiblen piezoresistiven Sensor (205) zu passen, und
eine Auslenkungswand (305), welche die Seitenwand abdeckt und geformt ist, um zwischen einen Radius und eine Flexor-Carpi-Radialis-Sehne zu passen, wobei die Auslenkungswand (305) zum Auslenken in Richtung des flexiblen piezoresistiven Sensors (205) proportional zu dem Druck konfiguriert ist, der durch eine radiale Arterie ausgeübt wird, wobei die Auslenkungswand (305) einen Federbereich (310) zwischen einem Benutzerschnittstellenabschnitt (308) der Auslenkungswand (305) und der Seitenwand (307) aufweist, wobei der Federbereich (310) die Auslenkungswand (307) in einem maximalen Abstand von dem flexiblen piezoresistiven Sensor (205) vorspannt, wenn kein Druck auf den Benutzerschnittstellenabschnitt (308) ausgeübt wird;
ein Druckumwandlungsmedium, das zwischen der einstückigen flexiblen Kappe (300) und dem flexiblen piezoresistiven Sensor (205) abgedichtet ist, sodass eine Auslenkung der Auslenkungswand (305) in Richtung des flexiblen piezoresistiven Sensors (205) eine proportionale Auslenkung des flexiblen piezoresistiven Sensors (205) bewirkt; und
eine starre Leitung (215) mit offenem Ende, die an dem Substrat (210) befestigt ist, die den flexiblen piezoresistiven Sensor (205) umgibt, wobei die starre Leitung (215) mit offenem Ende konfiguriert ist, um bündig in die Seitenwand (307) zu passen.

2. System nach Anspruch 1, wobei der Benutzerschnittstellenabschnitt (308) senkrecht zu der Seitenwand (307) ist.

3. System nach Anspruch 2, wobei der Federbereich (310) in Bezug auf den flexiblen piezoresistiven Sensor (205) ausgelenkt ist und von der Seitenwand (307) zum Benutzerschnittstellenabschnitt (308) im Durchmesser abnimmt.

4. System nach Anspruch 1, weiter umfassend:
ein Druckumwandlungskissen (705), das vorübergehend an der Haut eines Benutzers zwischen einem Radius des Benutzers und einer Flexor-Carpi-Radialis-Sehne des Benutzers angebracht werden kann und einen größeren Oberflächenbereich als der Benutzerschnittstellenabschnitt (310) der Auslenkungswand (305) aufweist, wobei das Druckumwandlungskissen (705) in Proportion zu dem Druck, der durch eine radiale Arterie ausgeübt wird, zum Auslenken nach außen von der Haut konfiguriert ist, sodass die Auswärtsauslenkung des Druckumwandlungskissens (705) eine proportionale Auslenkung der Auslenkungswand (305) in Richtung des flexiblen piezoresistiven Sensors (205) bewirkt, wenn der Benutzerschnittstellenabschnitt (308) in Kontakt mit dem Druckumwandlungskissen (705) angeordnet wird.

5. Verfahren zum Erfassen des arteriellen Drucks, umfassend:
Anbringen einer flexiblen Kappe (300) an Gewebe eines Benutzers über eine darunter liegende Arterie, sodass die flexible Kappe (300) in Reaktion auf einen Blutfluss durch die darunter liegende Arterie in einen abgedichteten Hohlraum (402) ausgelenkt wird;
über die flexible Kappe (300), Anlegen eines Drucks, der proportional zum arteriellen Blutfluss ist, auf eine feste Menge eines Druckumwandlungsmediums, das in dem abgedichteten Hohlraum (402) auf einer ersten Seite eines flexiblen piezoresistiven Sensors (215) angeordnet ist; und,
über das Druckumwandlungsmedium, Übertragen von Druckänderungen auf den flexiblen piezoresistiven Sensor (205), wobei der flexible piezoresistive Sensor (205) konfiguriert ist, um einen Innenwiderstand als Reaktion auf den Druck, der auf die erste Seite des flexiblen piezoresistiven Sensors (205) ausgeübt wird, zu verändern, wobei die flexible Kappe (300) umfasst:
eine Seitenwand (307), die bemessen ist, um um den flexiblen piezoresistiven Sensor (205) zu passen, und
eine Auslenkungswand (305), welche die Seitenwand (307) abdeckt und geformt ist, um zwischen einen Radius und eine Flexor-Carpi-Radialis-Sehne zu passen, wobei die Auslenkungswand (305) zum Auslenken in Richtung des flexiblen piezoresistiven Sensors (205) proportional zu Druckveränderungen in einer radialen Arterie ist, wobei die Auslenkungswand (305) einen Federbereich (310) zwischen einem Benutzerschnittstellenabschnitt (308) der Auslenkungswand (305) und der Seitenwand (307) aufweist, wobei der Federbereich (310) die Auslenkungswand (305) in einem maximalen Abstand von dem flexiblen piezoresistiven Sensor (205) vorspannt, wenn kein Druck auf den Benutzerschnittstellenabschnitt (308) ausgeübt wird,
wobei ein Substrat (210) an einer zweiten, gegenüberliegenden Seite des flexiblen piezoresistiven Sensors (205) befestigt ist, und wobei eine starre Leitung (215) mit offenem Ende an dem Substrat (210) befestigt ist, die den flexiblen piezoresistiven Sensor (205) umgibt, sodass Druck, der auf den flexiblen piezoresistiven Sensor (205) ausgeübt wird, bewirkt, dass der flexible piezoresistive Sensor (205) in einen Auslenkungshohlraum (315, 402) auslenkt, der proportional zu dem angelegten Druck ist, wobei der Auslenkungshohlraum (315, 402) auf einer zweiten Seite des flexiblen piezoresistiven Sensors (205) angeordnet ist, wobei die starre Leitung (315) mit offenem Ende konfiguriert ist, um bündig in die Seitenwand (307) zu passen.

6. Verfahren nach Anspruch 5, wobei der Federbereich (310) in Bezug auf den flexiblen piezoresistiven Sensor (205) ausgelenkt ist und von der Seitenwand (307) zum Benutzerschnittstellenabschnitt (310) im Durchmesser abnimmt.

7. Verfahren nach Anspruch 5, wobei der Benutzerschnittstellenabschnitt (308) senkrecht zur Seitenwand (307) ist.

8. Verfahren nach Anspruch 5, weiter umfassend:
ein Druckumwandlungskissen (705), das vorübergehend an der Haut eines Benutzers zwischen einem Radius des Benutzers und einer Flexor-Carpi-Radialis-Sehne des Benutzers angebracht werden kann und einen größeren Oberflächenbereich als der Benutzerschnittstellenabschnitt der Auslenkungswand (305) aufweist, wobei das Druckumwandlungskissen (705) zum Auslenken auswärts von der Haut in Proportion zu dem Druck konfiguriert ist, der durch eine radiale Arterie ausgeübt wird, sodass die Auswärtsauslenkung des Druckumwandlungskissens (705) einen proportionalen Druck zum Ausüben auf die Auslenkungswand (305) bewirkt, wenn der Benutzerschnittstellenabschnitt (310) in Kontakt mit dem Druckumwandlungskissen (705) angeordnet ist.

## Revendications

1. Système (200) de transduction de pression artérielle, comprenant :
un capteur piézorésistif souple (205) configuré pour modifier une résistance interne en fonction d'une déviation du capteur piézorésistif souple (205), dans lequel le capteur piézorésistif souple (205) est fixé à un substrat (210) de sorte que le capteur piézorésistif souple (205) dévie dans une cavité de déviation (315, 402) à la suite de la pression appliquée à une partie d'interface utilisateur (308) ;
un chapeau souple d'une pièce (300) comprenant :
une paroi latérale (307) calibrée pour s'ajuster autour du capteur piézorésistif souple (205) et
une paroi de déviation (305) coiffant la paroi latérale et moulée pour se conformer entre un radius et un tendon de muscle grand palmaire, la paroi de déviation (305) étant configurée pour dévier vers le capteur piézorésistif souple (205) en proportion de la pression appliquée par une artère radiale, dans lequel la paroi de déviation (305) comprend une région élastique (310) entre une partie d'interface utilisateur (308) de la paroi de déviation (305) et la paroi latérale (307), la région élastique (310) pressant la paroi de déviation (307) à une distance maximale du capteur piézorésistif souple (205) lorsqu'aucune pression n'est appliquée à la partie de l'interface utilisateur (308) ;
un milieu de transduction de pression scellé entre le chapeau souple d'une pièce (300) et le capteur piézorésistif souple (205) de sorte que la déviation de la paroi de déviation (305) vers le capteur piézorésistif souple (205) provoque une déviation proportionnelle du capteur piézorésistif souple (205) ; et
un conduit rigide à extrémités ouvertes (215) fixé au substrat (210) entourant le capteur piézorésistif souple (205), le conduit rigide à extrémités ouvertes (215) étant configuré pour s'ajuster de niveau à l'intérieur de la paroi latérale (307).

2. Système selon la revendication 1, dans lequel la partie d'interface utilisateur (308) est perpendiculaire à la paroi latérale (307).

3. Système selon la revendication 2, dans lequel la région élastique (310) est inclinée par rapport au capteur piézorésistif souple (205) et diminue en diamètre de la paroi latérale (307) à la partie d'interface utilisateur (308).

4. Système selon la revendication 1, comprenant en outre :
une plaquette de transduction de pression (705) qui peut être fixée de manière temporaire à la peau d'un utilisateur entre un radius de l'utilisateur et un tendon de muscle grand palmaire de l'utilisateur et ayant une surface spécifique supérieure à celle de la partie d'interface utilisateur (310) de la paroi de déviation (305), la plaquette de transduction de pression (705) étant configurée pour dévier vers l'extérieur de la peau en proportion de la pression appliquée par une artère radiale de sorte que la déviation vers l'extérieur de la plaquette de transduction de pression (705) provoque une déviation proportionnelle de la paroi de déviation (305) vers le capteur piézorésistif souple (205) lorsque la partie d'interface utilisateur (308) est placée en contact avec la plaquette de transduction de pression (705).

5. Procédé de détection d'une pression artérielle, comprenant :
l'application d'un chapeau souple (300) à un tissu d'un utilisateur au-dessus d'une artère sous-jacente de sorte que le chapeau souple (300) dévie dans une cavité scellée (402) à la suite de l'écoulement de sang à travers l'artère sous-jacente ;
via le chapeau souple (300), l'application d'une pression proportionnée à l'écoulement de sang artériel à une quantité fixe d'un milieu de transduction de pression situé dans la cavité scellée (402) sur un premier côté d'un capteur piézorésistif souple (215) ; et,
via le milieu de transduction de pression, la transmission de changements de pression au capteur piézorésistif souple (205), le capteur piézorésistif souple (205) étant configuré pour modifier une résistance interne à la suite de la pression appliquée au premier côté du capteur piézorésistif souple (205), dans lequel le chapeau souple (300) comprend :
un paroi latérale (307) calibrée pour s'ajuster autour du capteur piézorésistif souple (205) et
une paroi de déviation (305) coiffant la paroi latérale (307) et moulée pour se conformer entre un radius et un tendon de muscle grand palmaire, la paroi de déviation (305) étant configurée pour dévier vers le capteur piézorésistif souple (205) en proportion de changements de pression dans une artère radiale, dans lequel la paroi de déviation (305) comprend une région élastique (310) entre une partie d'interface utilisateur (308) de la paroi de déviation (305) et la paroi latérale (307), la région élastique (310) pressant la paroi de déviation (305) à une distance maximale du capteur piézorésistif souple (205) lorsqu'aucune pression n'est appliquée à la partie d'interface utilisateur (308),
dans lequel un substrat (210) est fixé à un second côté opposé du capteur piézorésistif souple (205) et dans lequel un conduit rigide à extrémité ouverte (215) est fixé au substrat (210) entourant le capteur piézorésistif souple (205) de sorte que la pression appliquée au capteur piézorésistif souple (205) amène le capteur piézorésistif souple (205) à dévier dans une cavité de déviation (315, 402) en proportion de la pression appliquée, la cavité de déviation (315, 402) étant située sur le second côté du capteur piézorésistif souple (205), le conduit rigide à extrémité ouverte (315) étant configuré pour s'adapter de niveau dans la paroi latérale (307).

6. Procédé selon la revendication 5, dans lequel la région élastique (310) est inclinée par rapport au capteur piézorésistif souple (205) et diminue en diamètre de la paroi latérale (307) à la partie d'interface utilisateur (310).

7. Procédé selon la revendication 5, dans lequel la partie d'interface utilisateur (308) est perpendiculaire à la paroi latérale (307).

8. Procédé selon la revendication 5, comprenant en outre :
une plaquette de transduction de pression (705) qui peut être fixée de manière temporaire à la peau d'un utilisateur entre un radius de l'utilisateur et un tendon de muscle grand palmaire de l'utilisateur et ayant une surface spécifique supérieure à celle de la partie d'interface utilisateur de la paroi de déviation (305), la plaquette de transduction de pression (705) étant configurée pour dévier vers l'extérieur depuis la peau en proportion de la pression appliquée par une artère radiale de sorte que la déviation vers l'extérieur de la plaquette de transduction de pression (705) amène une pression proportionnelle à s'appliquer à la paroi de déviation (305) lorsque la partie d'interface utilisateur (310) est en contact avec la plaquette de transduction de pression (705).
